# EUROPÄISCHE PATENTSCHRIFT

(11) **EP 0 811 012 B1**
(45) Veröffentlichungstag und Bekanntmachung des Hinweises auf die Patenterteilung: **26.05.1999**
(21) Anmeldenummer: 96902957.8
(22) Anmeldetag: 31.01.1996
(51) Int. Cl.: C07K 5/06

(54) **VERFAHREN ZUR HERSTELLUNG VON L-ASPARTYL-D-ALANIN-N-(THIETAN-3-YL)-AMIDEN**
METHOD OF PRODUCING L-ASPARTYL-D-ALANINE-N-(THIETHANE-3-YL)-AMIDES
PROCEDE DE PREPARATION DE L-ASPARTYL-D-ALANINE-N-(THIETANE-3-YL)-AMIDES

(30) Priorität: 21.02.1995 DE 19505933
(43) Veröffentlichungstag der Anmeldung: 10.12.1997
(73) Patentinhaber: Degussa Aktiengesellschaft, 60311 Frankfurt (DE)
(72) Erfinder: DRAUZ, Karlheinz, D-63579 Freigericht (DE); KNAUP, Günter, D-63486 Bruchköbel (DE); RETZOW, Stefan, D-63755 Alzenau (DE); SCHWARM, Michael, D-63755 Alzenau (DE); WECKBECKER, Christoph, D-63457 Hanau (DE)
(86) Internationale Anmeldenummer: EP9600393
(87) Internationale Veröffentlichungsnummer: WO9626213

(56) Entgegenhaltungen:
- EP-A- 0 034 876
- AT-B- 394 854
- CHEMICAL ABSTRACTS, vol. 81, no. 19, 11.November 1974 Columbus, Ohio, US; abstract no. 121026u, T NAGASE & F MASUKO: "Alpha-L-Asparaginyl-L-amino acid esters" Seite 578; XP002003940 & JP,A,07 435 352 (SUMITOMO ) 1.April 1974
- CHEMICAL ABSTRACTS, vol. 114, no. 3, 21.Januar 1991 Columbus, Ohio, US; abstract no. 24570j, K BURGER & M RUDOLPH: "Regiospecific reaction with omega-carboxy alpha-amino acids. A simple synthesis of aspartame" Seite 765; XP002003941 & CHEM. ZEITUNG, Bd. 114, Nr. 7-8, 1990, Seiten 249-251, in der Anmeldung erwähnt

## Beschreibung

Die Erfindung betrifft ein Verfahren zur Herstellung von L-Aspartyl-D-alanin-N-(thietan-3-yl)-amiden der allgemeinen Formel I 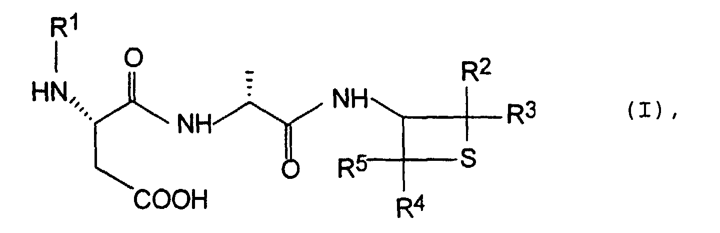 worin
R¹ für H oder eine selektiv abspaltbare Schutzgruppe steht und
R² - R⁵ unabhängig voneinander gleich oder verschieden H oder C₁-C₄-Alkyl, linear oder verzweigt, bedeuten.

Verbindungen mit R¹ = H sind Dipeptid-Süßstoffe (US-PS 4,411,925, EP-A 34 876). Alitam (R¹ = H, R² - R⁵ = CH₃) hat eine Süßkraft, die etwa 2000 mal so groß ist wie die des Zuckers (ACS Symp. Ser. 1992, 450, 57).

Die Verbindungen der Formel I werden vorzugsweise ausgehend von Thietanaminen, D-Ala und L-Asp hergestellt.

Diese Komponenten können grundsätzlich auf verschiedene Weise miteinander gekoppelt werden. So wird die Synthese von Verbindungen der allgemeinen Formel I beispielsweise in der US-PS 4,411,925 beschrieben. Die Synthesen der drei Kopplungspartner (Thietanamin und aktivierte/geschützte D-Ala und L-Asp) erfolgen dort jedoch auf recht umständliche Weise nach literaturbekannten Verfahren. Dies wird am Beispiel des Alitams verdeutlicht.

Aus Diisopropylketon wird nach Dibromierung durch Zyklisierung mit Dinatriumsulfid das 2,2,4,4-Tetramethyl-3-thietanon hergestellt, welches im System Ammoniumacetat/Natriumcyanoborhydrid in 42 % Ausbeute zum Thietanamin reduziert wird. Das Amin kann z. B. nach EP-A 168 112 auch durch Überführen des 2,2,4,4-Tetramethylthietanons in ein Oxim (< 30 % Ausbeute) und Reduktion mittels Lithiumaluminiumhydrid (42 % Ausbeute) gewonnen werden. In US-PS 4,851,548 wird weiterhin die Reduktion des 2,2,4,4-Tetramethyl-3-thietanons zum Thietanamin mittels Leuckart-Wallach Reaktion in max. 65 % Ausbeute beschrieben.

Für die Kopplung der drei Reaktionspartner sind in US-PS 4,411,925 verschiedene Strategien angegeben, welche nach allgemein gängigen Peptidkopplungsmethoden mit entsprechend vielen Schutz- und Entschützungsschritten vonstatten gehen. So wird für die selektive α-Kupplung der L-Asp ein relativ teurer und schwierig zu synthetisierender N-Benzyloxycarbonyl-L-Asparginsäure-β-benzylester verwandt, der nach Aktivierung mit D-Ala-thietanamid umgesetzt wird. Eine andere dort angegebene Vorschrift benutzt als aktivierte und zugleich geschützte L-Asp das N-Thiocarboxyanhydrid. Dieses ist schwierig und nur in geringen Ausbeuten zu synthetisieren (42 % Ausbeute). Zudem werden hinsichtlich Arbeitsschutz und Umweltbelastung kritische Materialien wie Methylenchlorid, Schwefelkohlenstoff und Phosphortribromid zu dessen Synthese benötigt.

Demgegenüber war es Aufgabe der Erfindung, ein Verfahren zur Herstellung von Verbindungen der allgemeinen Formel I, insbesondere aber von Alitam, anzugeben, das gegenüber den bekannten Verfahren eine erhebliche Verbesserung in Methodik sowie Ausbeute und Zeitaufwand bedeutet.

Gelöst wird diese Aufgabe durch Umsetzung von D-Alanin-thietanamiden der allgemeinen Formel II 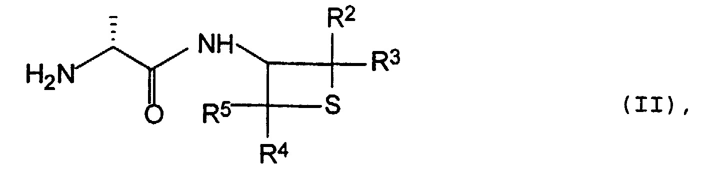 worin
R² - R⁵ die bei Formel (I) angegebene Bedeutung besitzen,
mit
Oxazolidinonverbindungen der allgemeinen Formel III 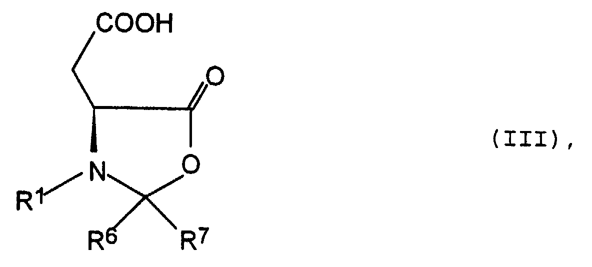 worin
R¹ die bei Formel I angegebene Bedeutung besitzt und R⁶ und R⁷ unabhängig voneinander gleich oder verschieden für H, C₁-C₄-Alkyl, linear oder verzweigt, Aryl oder einen die Carbonylgruppe aktivierenden, bevorzugt halogensubstituierten Rest stehen, in einem inerten organischen Lösungsmittel.

1,3-Oxazolidin-5-one und ihre prinzipielle Eignung zur Peptidkopplung sind grundsätzlich bekannt (J. P. Greenstein, M. Winitz, Chemistry of the Amino Acids, Wiley, 1961, S. 1024; Houben-Weyl, Bd. 15/I, S. 91f).

Trotz ihrer einfachen Zugänglichkeit und im Falle der Asparaginsäure hohen Regioselektivität haben sie bisher keine breite Anwendung zur Herstellung von Asparginsäurepeptiden gefunden. Die Gründe dafür sind, daß die Meinung bestand, daß die zur Herstellung im Überschuß einzusetzende bzw. bei der Reaktion freigesetzte Carbonylverbindung zu unerwünschten Nebenreaktionen mit der zur Kopplung eingesetzen Aminkomponente führt, und daher eine aufwendige Reinigung und Isolierung unumgänglich sei (Greenstein, Winitz, S. 1027, Houben-Weyl, Bd. 15/II, S. 95).

Da weiterhin die zur Herstellung von Verbindungen der Formel (I) einzusetzende Aminkomponente der Formel (II) mit dem Thietanring eine weitere, sehr reaktive funktionelle Gruppe besitzt (Comprehensive Heterocyclic Chemistry, Vol. 7, S. 419f), war es sehr überraschend, daß die Verbindungen der Formel (I) mit dem erfindungsgemäßen Verfahren in guten Ausbeuten auf einfachem Weg herstellbar sind.

Obwohl Oxazolidinone zur Herstellung von Aspartam geeignet sind (Chemiker-Ztg. 1990, 114, 249; US-PS 4,730,076; JP-A-7435352), wurden sie trotzdem - u. a. aus den o. g. Gründen - bisher nicht im technischen Maßstab zur Synthese von Aspartam eingesetzt. Weitere Gründe hierfür können z. T. Unwirtschaftlichkeit (Boc-Oxazolidinone, Oxazolidinone aus Hexafluoraceton, Teoc-Oxazolidinone, Tosyl-Oxazolidinone) und z. T. fehlende Abspaltungsmöglichkeiten der Schutzgruppen (Moc-Oxazolidinone) sein.

Benutzt man sehr reaktive Carbonylkomponenten als Oxazolidinonbildner, dann kann auf den Schutz der Aminofunktion der Aminosäure verzichtet werden. Aktive Carbonylkomponenten sind beispielsweise alle Aldehyde und Ketone, die leicht Hydrate bilden.

In vorteilhafter erfindungsgemäßer Verfahrensabwandlung wird eine Verbindung der Formel III mit R¹ = H, R⁷ = CF₃ und R⁶ = CF₃ eingesetzt, die durch Umsetzung von L-Asparaginsäure mit Hexafluoraceton erhalten wurde.

Bei der Verbindung der allgemeinen Formel III mit R₁ = H, R₆ und R₇ = CF₃ handelt es sich um das Bis-(2,2-trifluor-4-carboxymethyl)-1,3-Oxazolidin-5-on, welches einen Vertreter der Oxazolidinone der L-Asp mit aktiver Carbonylkomponente darstellt.

Diese Verbindung ist bereits bekannt (Chem. Ber. 1964, 99, 1461) und wurde bereits für Peptidkopplungen eingesetzt. Bei der Herstellung des Oxazolidinons wurde jedoch immer DMSO als Lösungsmittel verwendet und das Oxazolidinon durch eine aufwendige, wäßrige Aufarbeitung isoliert. Das schwerflüchtige DMSO und der zur Bindung des Reaktionswassers erforderliche Überschuß Hexafluoraceton werden dabei mit Wasser extrahiert, wodurch eine Recyclierung des DMSOs und des Hexafluoracetons nahezu ausgeschlossen ist. Völlig überraschend wurde gefunden, daß man die Umsetzung von L-Asp mit Hexafluoraceton in besonders vorteilhafter Weise in DMF als Lösungsmittel vollziehen kann und daß das überschüssige Hexafluoraceton und/oder Hexafluoracetonhydrat vollständig zurückgewonnen werden kann. Dazu wird Hexafluoraceton-Trihydrat nach Dehydrierung in eine Suspension von L-Asp in DMF eingeleitet. Nach Einleiten von 2,5 bis 3 Äquiv. Hexafluoraceton wird solange gerührt, bis alle L-Asp in Lösung gegangen ist. Nach vollendeter Reaktion wird zuerst Hexafluoracetonhydrat im Vakuum entfernt und anschließend das Lösungsmittel. Beide Verbindungen sind dadurch der Recyclierung zugänglich.

Das gewünschte Oxazolidinon (R₁ = H, R_{6,7} = CF₃) fällt als farbloses Öl an und kann besonders vorteilhaft ohne weitere Reinigung oder Isolierung in die folgende Kopplungsreaktion eingesetzt werden.

Nach vollzogener Kopplung mit D-Ala-thietanamid, die bereits bei RT hinreichend schnell und in > 80 % Ausbeute abläuft, wird das freiwerdende Hexafluoraceton im Vakuum entfernt und ebenfalls aufgefangen. Es können so 85 % des eingesetzten toxischen und umweltbelastenden Hexafluoracetons recycliert werden. Die verbleibende Lösung wird eingeengt und kann, wie z. B. in US-PS 4,411,925 beschrieben, aufgearbeitet werden.

Ein weiterer Vertreter der Oxazolidinone der L-Asp mit aktiver Carbonylkomponente ist eine Verbindung der allgemeinen Formel III mit R₁ = H, R⁶ = CCl₃ und R₇ = H. Diese ist im Rahmen der Erfindung durch Umsetzung von L-Asparaginsäure mit Chloral erhältlich.

Die Darstellung von Oxazolidinonen aus Aminosäuren und Chloral wird zwar bereits in Angew. Chem. 1959, 71, 339 beschrieben, nicht jedoch die Synthese ausgehend von L-Asp und Chloral. In AT 394 854 wird das Oxazolidinon aus Asp und Chloral erwähnt. Zur Umsetzung mit Aminen ist laut dieser Literaturstelle aber das Acyl-geschützte Oxazolidinon erforderlich. Es wurde nun überraschend gefunden, daß eine Verbindung der Formel III mit R₁ = H und R₆ oder R₇ = CCl₃ zur Kopplung mit dem D-Ala-thietanamid der allgemeinen Formel II eingesetzt werden kann, ohne daß die N-Funktion durch eine Acylgruppe geschützt werden muß und daß das Oxazolidinon nicht isoliert werden muß. Erfindungsgemäß geht man am besten so vor, daß eine Suspension von L-Asp in wenig DMSO mit einem Überschuß Chloral - vorzugsweise 2 Äquiv. - versetzt und mehrere Stunden - vorzugsweise 4 bis 6 h bei RT gerührt wird. Dann wird die Lösung mit einem indifferenten org. Lösungsmittel - vorzugsweise einem Ether, besonders bevorzugt ist THF - versetzt und bei Temperaturen unter 0 °C - vorzugsweise -15 °C - mit einem Überschuß der Aminkomponente (allgemeine Formel II) gerührt. Nach mehreren Stunden Reaktionszeit - vorzugsweise 12 Stunden - wird durch Filtration aufgearbeitet. Nach säulenchromatographischer Reinigung erhält man das Kopplungsprodukt (R₁ = H) in > 80 % Ausbeute.

Als N-geschützte Aminosäurekomponenten zur Oxazolidinonbildung kommen im Rahmen der Erfindung bevorzugt auch alle aus der Peptidchemie bekannten N-acyl-, -alkyloxycarbonyl, -arylalkyloxycarbonyl- sowie heterosubstituierte -alkyl- und -arylalkyloxycarbonyl geschützten L-Asparaginsäuren in Frage.

In einer weiteren erfindungsgemäß bevorzugten Verfahrensvariante werden daher zur Kopplung mit D-Ala-thietanamid Verbindungen der Formel III eingesetzt, in der R¹ für eine N-Schutzgruppe steht, wobei die Verbindung der Formel III durch Umsetzung von N-geschützter L-Asparaginsäure der allgemeinen Formel IV 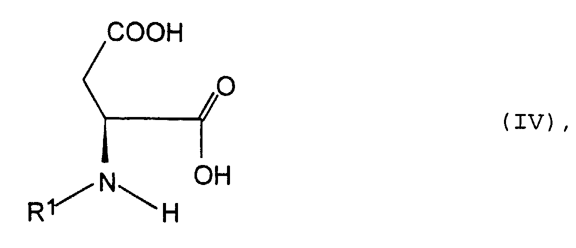 worin
R¹ für Boc, Z, TFA, Aloc, Teoc, Formyl, Tosyl, Fmoc oder Moc steht,
mit Carbonylverbindungen, vorzugsweise Aldehyden, besonders bevorzugt Formaldehyd oder einer bei der Umsetzung Formaldehyd erzeugenden Vorstufe, erhalten wird und wobei das Umsetzungsprodukt aus der Verbindung der Formel IV mit Formaldehyd oder einer Vorstufe davon, wie Paraformaldehyd oder Trioxan, ohne Isolierung und Aufreinigung zur Kopplung mit der Verbindung der allgemeinen Formel II eingesetzt wird.

Die Verbindungen der allgemeinen Formel III, in denen R¹ nicht für H steht und für die gilt
- R₁ =: Z, R₆, R₇ = H, (Chem. Pharm. Bull. 1969, 17, 1679);
- R₁ =: Boc, R_{6,7} = H (J. Polym. Sci., Polym. Chem. Ed. 1978, 16, 2237);
- R₁ =: Teoc, R_{6,7} = H (Bull. Chem. Soc. Jpn. 1982, 55, 633);
- R₁ =: Fmoc, R_{6,7} = H (Chimia 1992, 46, 314);
- R₁ =: Tosyl, R_{6,7} = H (Chem. Ber. 1962, 95, 1009);
- R₁ =: Moc, R_{6,7} = H (THL 1992, 33, 2669); und
- R₁ =: Alloc, R_{6,7} = H (Synthesis 1991, 935),
sind bekannt.

Neu sind die Verbindungen, in denen R¹ für TFA oder Formyl steht.

Die Abspaltung der N-Schutzgruppen zum Erhalt der ungeschützten Verbindung der Formel I kann nach literaturbekannten Verfahren erfolgen. Diese sind beispielsweise in Lehrbüchern wie Houben-Weyl Band 15/1 beschrieben.

Erfindungsgemäß werden mit besonderem Vorteil als Verbindung der Formel IV die mit Boc oder Formyl N-geschützte L-Asparaginsäure eingesetzt.

Die Synthese der Verbindungen der allgemeinen Formel III und deren Umsetzung zu Verbindungen der Formel I mit Verbindungen der Formel II wird am besten analog zur nachfolgend beschriebenen Verfahrensweise für das Z-Oxazolidinon durchgeführt. Z-L-Asp wird mit Paraformaldehyd in einem organischen Lösungsmittel, bevorzugt Toluol oder Methylisobutylketon, zum entsprechenden 1,3-Oxazolidinon umgesetzt. Dieses wird dann mit 1 Äquiv. der Aminkomponente der allgemeinen Formel II in Gegenwart einer Hilfsbase, wie z. B. einem tert. Amin, z. B. Triethylamin, bei erhöhter Temperatur - vorzugsweise 60 °C - mehrere Stunden - vorzugsweise 6 - gerührt. Man arbeitet auf und erhält das Z-Kopplungsprodukt in 85 % Ausbeute. Die Abspaltung der Z-Gruppe kann nicht durch katalytische Hydrierung geschehen, da der im Molekül vorhandene Schwefel den Katalysator inhibiert. Die Entfernung der Schutzgruppe erfolgt daher im System Eisessig/HBr. Der erhaltene Rückstand wird entsprechend US-PS 4,411,925 aufgearbeitet.

Die Kopplungsprodukte von Oxazolidinonen der allgemeinen Formel III, die eine andere der oben genannten Schutzgruppen tragen, und Verbindungen der allgemeinen Formel II werden nach Standardmethoden entschützt.

Bei Einsatz der Boc- oder Formyl-Schutzgruppe, die erfindungsgemäß bevorzugt sind, gestaltet sich demnach die Abspaltung einfacher.

Im Rahmen des erfindungsgemäßen Verfahrens werden die Verbindungen der allgemeinen Formel II nach literaturbekannten Verfahren hergestellt (US-PS 4,411,925). Bevorzugt werden dabei die Verbindungen der allgemeinen Formel II durch Kopplung von D-Alanin-Verbindungen der allgemeinen Formel V 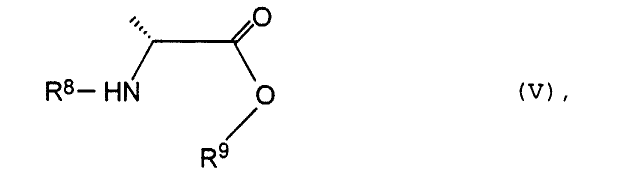 worin
R⁸ eine selektiv abspaltbare N-Schutzgruppe bedeutet und R⁹ ein aus der Peptidchemie bekannter, die Carbonylgruppe aktivierender Rest ist,
mit
Thietanaminen der allgemeinen Formel VI 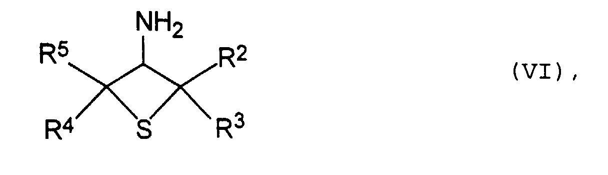 worin
R² - R⁵ die bei Formel I angegebene Bedeutung besitzen, erhalten.

Weitere Einzelheiten sind der gesamten US-PS 4,411,925 entnehmbar.

Erfindungsgemäß werden die Thietanamine der allgemeinen Formel VI bevorzugt durch Kondensation von Thietanonen der allgemeinen Formel VII 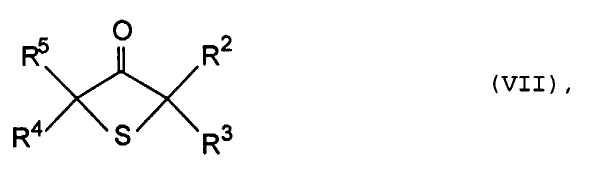 worin
R² - R⁵ die bei Formel I angegebene Bedeutung besitzen, mit Aminderivaten der allgemeinen Formel VIII

   NH₂-X (VIII),

   worin X für OH, O-Alkyl, O-Aryl, O-Acyl oder NHR¹⁰ steht und R¹⁰ = C₁-C₄-Alkyl, Aryl, Aralkyl, Tosyl, Mesyl oder Acyl ist, oder deren Salze, wie beispielsweise Hydrohalogenide, Phosphate, Citrate, Acetate oder Sulfate,
   zu Thietaniminen der allgemeinen Formel IX 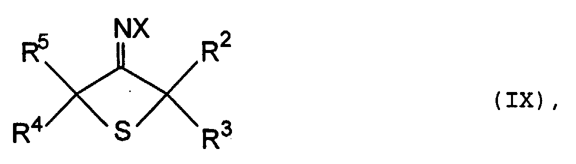 worin
R² - R⁵ die bei Formel I angegebene Bedeutung besitzen und X die Bedeutung wie in Formel (VIII) hat,
und anschließende Reduktion der erhaltenen
Thietaniminderivate,
erhalten.

Reaktionen zur Darstellung von Thietaniminen der allgemeinen Formel IX (R² - R⁵ = Methyl) werden in Chem. Ber. 1991, 124, 1747 (Hydrazon: 97 % Ausbeute, 93 h Reaktionszeit) und in Tetrahedron 1986, 42, 5301 (Tosylhydrazon: 38 % Ausbeute, 4 d Reaktionszeit) beschrieben.

In US-PS 4,692,512 und EP-A 168 112 wird das entsprechende Oxim (X = OH, R² - R⁵ = Methyl) synthetisiert. Die dort angegebene Reaktionsvorschrift bezieht sich auf eine Prozedur aus US-PS 4,411,925. Folgt man der dort angegebenen Vorschrift zur Synthese des Oxims, erhält man dieses allerdings nur mit < 30 % Ausbeute. Das erhaltene Oxim wird dann mit einem großen Überschuß (5 Hydrid-Äquivalente) Lithiumaluminiumhydrid in nur 35 %iger Ausbeute zum Amin reduziert.

Thietanone der allgemeinen Formel VII (R² - R⁵ = Methyl) können laut US-PS 4,411,925, EP-A 34 876, US-PS 4,399,163, EP-A 69 811, US-PS 4,517,379 mit einem großen Überschuß Ammoniumacetat und Natriumcyanoborhydrid in 42 % Ausbeute zum Amin reduziert werden.

Ebenfalls gelingt laut US-PS 4,851,548 eine Leuckart-Wallach Reduktion des Thietanons der allgemeinen Formel VII (R² - R⁵ = Methyl) mit einem großen Überschuß Formamid in max. 65 %iger Ausbeute.

Im allgemeinen kann die Reduktion von Iminderivaten durch heterogene katalytische Hydrierung, durch Borane, mittels Lithiumaluminiumhydrid, durch Alkalimetalle in Ammoniak, Zink in Eisessig, enzymatisch oder elektrochemisch erfolgen (Houben-Weyl Band E16d, Teil 2).

Nachteilig bei den bekannten Verfahren ist grundsätzlich die schlechte Ausbeute, die eine wirtschaftliche Herstellung von Verbindungen der allgemeinen Formel VI bislang nicht zuließ. So lassen sich die Iminderivate der allgemeinen Formel IX gemäß dem Stand der Technik nur in schlechten Ausbeuten oder extrem langen Reaktionszeiten darstellen, während die bekannten Reduktionsverfahren in jedem Falle schlechte Ausbeuten liefern und große Überschüsse des reduzierenden Agens erfordern oder im technischen Maßstab nur sehr schwer durchführbar sind. U. a. werden Iminderivate vorzugsweise im großen Maßstab mittels heterogener katalytischer Hydrierung reduziert. Dieses Verfahren kann auf Imine der allgemeinen Formel IX jedoch nicht angewendet werden, da der im Molekül vorhandene Schwefel den Katalysator inhibiert.

Dadurch, daß erfindungsgemäß die Kondensation des Thietanons der allgemeinen Formel VII mit dem Amin der allgemeinen Formel VIII oder dessen Salz im Autoklaven bei einer Temperatur > 80 °C, bevorzugt > 100 °C, in Gegenwart eines Salzes einer Säure, deren pKs-Wert im Bereich zwischen 4 und 1 liegt, und die sich anschließende Reduktion mit Natriumborhydrid oder Lithiumborhydrid und einem Aktivator durchgeführt werden, gelingt es äußerst vorteilhaft, die bislang nicht in befriedigender Ausbeute erhältlichen Thietanamine der allgemeinen Formel VI in hohen Ausbeuten zur Verfügung zu stellen (> 90 % Ausbeute für die Kondensationsreaktion bezogen auf das Thietanon der allgemeinen Formel VII und auch > 90 % Ausbeute für die Reduktionsreaktion bezogen auf das Thietaniminderivat der allgemeinen Formel IX).

Somit ergeben sich für das erfindungsgemäße Verfahren ausgehend vom jeweiligen Thietanon Ausbeuten von > 80 % am entsprechenden Thietanamin. Diese nicht ohne weiteres vorhersehbare Steigerung ist mit ursächlich für die Entwicklung eines wirtschaftlich interessanten Verfahrens zur Herstellung der Dipeptid-Süßstoffe der allgemeinen Formel I.

Wie aus dem Stand der Technik bekannt, ließen sich Thietaniminderivate der allgemeine Formel IX bislang nur in äußerst schlechten Ausbeuten oder durch extrem lange Reaktionszeiten darstellen.

Erfindungsgemäß wurde nun unerwartet gefunden, daß sich Thietanimin-Derivate der allgemeinen Formel IX in sehr guten Raum/Zeit-Ausbeuten darstellen lassen, wenn man zu deren Herstellung einen geschlossenen Autoklaven bei hoher Temperatur und gleichzeitigem Zusatz einer Base einer schwachen Säure, vorzugsweise eines Alkali-Salzes der Phosphorsäure, verwendet.

Hierbei richtet sich die anzuwendende Temperatur genauso wie der Zusatz der Hilfsbase nach dem umzusetzenden Thietanon-Derivat.

Die Temperatur sollte möglichst hoch sein, damit eine schnelle Umsetzung erfolgt. Andererseits kann es bei hohen Temperaturen zur Bildung von Nebenprodukten kommen, vor allem wenn die Säurestärke der eingesetzten Säure zu hoch ist. Dadurch, daß der pKs-Wert der verwendeten zur Hilfsbase konjugierten Säure zwischen 4 und 1 liegt, wird erreicht, daß die Nebenproduktbildung bei einer hinreichend schnellen Reaktion völlig unterdrückt wird, wobei es offenbar gelingt, eine Zersetzung des Thietanringes zu vermeiden. Dies ist überraschend und nicht vorhersehbar gewesen.

Im Rahmen der Erfindung sind für die Kondensationsreaktion zwischen Thietanonen der allgemeinen Formel VII und Aminderivaten der allgemeinen Formel VIII im geschlossenen Autoklaven Temperaturen von > 80 °C erforderlich, von > 100 °C bevorzugt und ganz besonders hohe Temperaturen von 120 °C oder darüber von Vorteil. Als einzusetzende Hilfsbase kommen Verbindungen in Frage, deren konjugierte Säure einen pKs-Wert im Bereich von 4 bis 1 aufweist, vorzugsweise Monoalkalisalze der Phosphorsäure, z. B. Kaliumdihydrogenphosphat. Zu anderen mit Vorteil einsetzbaren Salzen gehören z. B. Natriumformiat, Natriumcitrat. In einer besonders bevorzugten erfindungsgemäßen Verfahrensabwandlung kann als Base einer schwachen Säure ein Säureadditionssalz des Amins der allgemeinen Formel VIII mit einer Säure eingesetzt werden, deren pKs-Wert zwischen 4 und 1 liegt. In diesem Fall kommt es mit fortschreitender Reaktion zur Freisetzung der schwachen Säure.

Erfindungsgemäß sind auch Mischformen denkbar, d. h. die Kondensation des Thietanons der allgemeinen Formel VII mit einem Amin der allgemeinen Formel VIII wird in Gegenwart einer Säure durchgeführt, wobei die Säure oder ein Gemisch von Säuren jeweils mit einem pKs von zwischen 4 und 1 über ein Gemisch von Hilfsbasen in die Reaktion eingeführt werden. Bei der Mischung von Hilfsbasen kann es sich hierbei um beispielsweise Alkalisalze der Phosphorsäure mit Säureadditionssalzen des Amins der allgemeinen Formel VIII handeln. In diesem Fall versteht es sich, daß zusätzlich auch noch eine bestimmte stöchiometrische Menge des freien Amins der allgemeinen Formel VIII oder eines seiner Salze einzusetzen ist.

Das in der Kondensationsreaktion gemäß der Erfindung erhaltene Thietanimin-Derivat kann im Rahmen der Erfindung völlig überraschend mit einem leicht zu handhabenden aktivierten Natrium- oder Lithiumborhydrid, wie in der japanischen Offenlegungsschrift Hei-221935 für die Reduktion von Aminosäuren zu Aminoalkoholen beschrieben, in sehr guten Ausbeuten (> 90 %) zum entsprechenden Thietanamin der allgemeinen Formel VI reduziert werden.

Grundsätzlich kommen dabei für den erfindungsgemäßen Reduktionsschritt alle dem Fachmann bekannten Aktivatoren in Frage. Zu den mit Vorteil einsetzbaren Aktivatoren gehören vor allem Jod, Chlorwasserstoff oder Schwefelsäure. Hiervon ist die Verwendung von Schwefelsäure oder Chlorwasserstoff als Aktivator für das Natrium- oder Lithiumborhydrid bevorzugt; der Einsatz von Schwefelsäure zur Aktivierung von Natriumborhydrid ist besonders vorteilhaft.

Weiterhin ist es von Vorteil für das Verfahren der Erfindung, wenn die Reduktionsreaktion in einem Lösungsmittel durchgeführt wird. Hierbei sind Lösungsmittel mit Etherstruktur, ggf. in Kombination mit anderen inerten Lösungsmitteln, besonders bevorzugt. Es sind Dialkylether, cyclische Ether, wie THF und Dioxan, sowie Ethylenglykolether, wie Dimethoxyethan, bevorzugt der höhersiedende Diethylenglykoldimethylether, geeignet. Ebenfalls bevorzugt sind Lösungsmittelgemische aus Ethern und anderen aprotischen Lösungsmitteln, wie z. B. Toluol, etc.

Die Reaktionstemperatur muß nach Zugabe der Schwefelsäure im allgemeinen > 80 °C, vorzugsweise über 100 °C, liegen. In bevorzugter erfindungsgemäßer Verfahrensmodifikation vollzieht sich der Reduktionsschritt bei 110 °C. Niedrigsiedende Ether werden deshalb unter erhöhtem Druck für die Reaktion eingesetzt.

Als ein mit besonderem Vorteil für den Reduktionsschritt der Erfindung anzuwendendes Reduktionssystem hat sich die Kombination von Natriumborhydrid mit Schwefelsäure als Aktivator erwiesen. Dieses System hat den Vorteil, daß die einzusetzenden reduzierenden und aktivierenden Agentien leicht handhabbar und relativ preiswert sowie äußerst wirksam sind.

Das erfindungsgemäße zweistufige Verfahren zur Herstellung von Thietanaminen der allgemeinen Formel VI aus Thietanonen der allgemeinen Formel VII hat sich als besonders vorteilhaft erwiesen, wenn als Thietanon der allgemeinen Formel VII eine Verbindung eingesetzt wird, in welcher die Reste R¹ - R⁴ jeweils für Methyl stehen und/oder wenn als Aminderivat der allgemeinen Formel VIII eine Verbindung gewählt wird, in der X für OH steht. Sofern die Aminderivate der allgemeinen Formel VIII in der Lage sind Salze zu bilden, versteht es sich, daß auch diese Salze zur Kondensation im Autoklaven verwendet werden können. Beispiele für Salze sind u. a. Hydroxylaminhydrochlorid oder Hydroxylamindihydrosulfat.

Verfahren zur Herstellung der als Zwischenprodukte im Verfahren der Erfindung Bedeutung besitzenden Thietanone der allgemeinen Formel VII sind schon literaturbekannt.

So ist aus Tetrahedron Lett. 1985, 26, 5187 eine Herstellungsvariante für eine Verbindung der allgemeinen Formel I mit R¹ - R⁴ = Methyl bekannt, bei der diese Verbindung über eine Carbenaddition an ein zyklisches Disulfid hergestellt wird. Allerdings sind die Ausbeuten bei dieser Variante äußerst schlecht.

Etwas bessere Ausbeuten erhält man bei der Herstellung von Verbindungen der allgemeinen Formel VII über eine Dibromierung von Ketonen der allgemeinen Formel XI 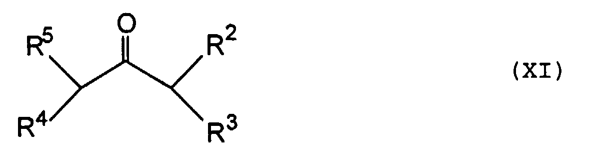 und anschließender Zyklisierung mit Schwefelwasserstoff in Gegenwart einer Base oder mit NaHS oder Na₂S (Arkiv Kemi 1963, 21, 295; J. Chem. Soc. Perkin Trans I, 1975, 2513, ibid 1976, 2590; J. Am. Chem. Soc. 1976, 98, 6696; Chem. Ber. 1980, 113, 2255; J. Med. Chem. 1990, 33, 1052; Chem. Ber. 1991, 124, 1747; J. Am. Chem. Soc. 1976, 98, 7081; Chem. Ber. 1984, 117, 277; J. Chem. Soc. Perkin Trans I 1984, 2457; Tetrahedron 1986, 42, 5301; US-PS 4,851,548).

Nachteilig an diesen bekannten Verfahren ist jedoch, daß Brom zum Einsatz kommt und eine Bromidverbindung als Abfallprodukt anfällt, welche aufgearbeitet oder entsprechend entsorgt werden muß. Ein weiterer Nachteil ist in der Verwendung des sehr toxischen Schwefelwasserstoffes zu sehen. Beide Aspekte sind hinsichtlich Arbeitsschutzmaßnahmen und Umweltbelastung kritisch.

Dadurch, daß im Rahmen der Erfindung β-Oxosulfenyle der allgemeinen Formel X 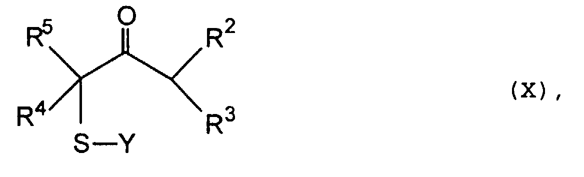 worin
R² - R⁵ die bei Formel I angegebene Bedeutung besitzen und
Y für Cl, Br, J steht, in einem organischen Lösungsmittel unter Einfluß einer Base zyklisiert werden, wurde es in nicht ohne weiteres vorhersehbarer Weise möglich, die in der Literatur angegebenen Ausbeuten deutlich zu erhöhen. Ferner gelang es besonders vorteilhaft, die Verwendung des allgemein sehr toxischen Schwefelwasserstoffes, welcher ansonsten zur Zyklisierung der Dibromverbindung der allgemeinen Formel (b) 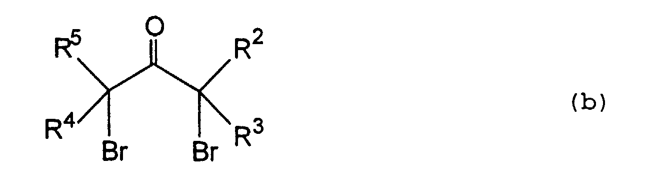
verwendet wird, zu vermeiden.

Dabei kann die Zyklisierung von Oxosulfenyl-Verbindungen der allgemeinen Formel X in inerten organischen Lösungsmitteln erfolgen. Zu den erfindungsgemäß bevorzugten Lösungsmitteln gehören beispielsweise Ether, wie beispielsweise THF, Diethylether, Diisopropylether oder Tertiärbutylether, (C₁-C₆) Alkohole, wie beispielsweise Methanol, Ethanol, Isopropanol, n-Propanol, tert.Butanol, n-Butanol, n-Pentanol oder n-Hexanol, sowie aliphatische oder aromatische Kohlenwasserstoffe, wie beispielsweise n-Hexan, Cyclohexan oder Toluol oder Gemische der geannten Lösungsmittel, und Alkane; ganz besonders bevorzugt sind im Rahmen der Erfindung Lösungsmittel wie Methanol, Ethanol, Isopropanol, Butanol und Tetrahydrofuran, Diethylether, Diisopropylether, Tertiärbutylmethylether.

Als Base für die Zyklisierungsreaktion lassen sich im Rahmen der Erfindung grundsätzlich eine große Reihe von Substanzen verwenden. Zu den bevorzugt einzusetzenden Verbindungen gehören Kaliumcarbonat, Natriumhydrid sowie Alkalialkoholate. Ganz besonders bevorzugt ist die Verwendung von KOtBu bei Einsatz von Ethern als Lösungsmittel sowie bei Verwendung von Alkoholen der Einsatz der entsprechenden Natriumalkoholate.

Die für die Zyklisierung zu Thietanonen benötigten beta- Oxosulfenylverbindungen der allgemeinen Formel II können nach literaturbekannten Verfahren herstellt werden. Aus Helv. Chim. Acta 1966, 49, 2344 und J. Prakt. Chem. 1979, 321, 1017 ist bekannt, daß sich Diisopropylketon mit Schwefeldichlorid zum entsprechenden Oxosulfenylchlorid der Formel (a) 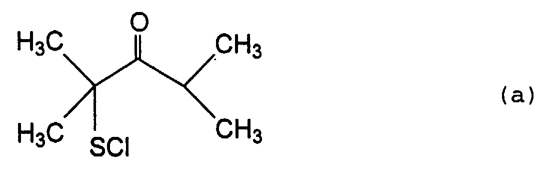 umsetzen läßt.

Dabei wird beispielsweise in Helv. Chim. Acta 1966, 49, 2344 vorgeschlagen, die Reaktion zwischen Schwefeldichlorid und Diisopropylketon durch Zutropfen von Schwefeldichlorid zu einer Lösung von Diisopropylketon in Chloroform bei 50 bis 60 °C mit etwas Aluminiumchlorid als Katalysator auszuführen. Gemäß der in dieser Fundstelle angegebenen Vorschrift wird das Reaktionsgemisch nach beendeter Chlorwasserstoff-Entwicklung zur Isolierung der Reaktionsprodukte fraktioniert destilliert. Hierbei wird das Oxosulfenylchlorid der Formel (a) lediglich in ca. 50 %iger Ausbeute erhalten.

Eine weitere Möglichkeit zur Herstellung der Sulfenylchloride der Formel (a) besteht in der Umsetzung von Diisopropylketon mit Dischwefeldichlorid zum Bisdiisopropyldisulfid (Lit.: Helv. Chim. Acta 1966, 49, 2344. Ausbeute: 55 %). Es ist weiterhin bekannt, daß Disulfide mit Chlor zu den entsprechenden Sulfenylchloriden umgesetzt werden können (Lit.: Houben Weyl "Methoden der organischen Chemie", Bd. Ell, S. 72ff).

β-Oxosulfenylverbindungen der Formel X lassen sich im Rahmen der Erfindung vorteilhafterweise durch Umsetzung von Ketonen der allgemeinen Formel XI 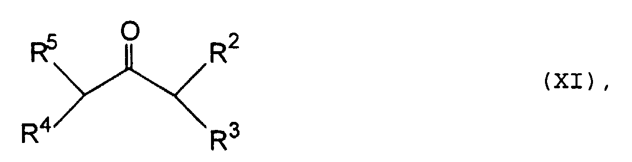 worin R² - R⁵ die bei Formel I angegebene Bedeutung besitzen, mit Schwefelverbindungen der allgemeinen Formel XII

SY₂ (XII),

worin Y für Cl, Br und/oder J steht, unter Ausschluß eines Zusatzes von Lösungsmittel und weiterer Lewis-Säuren erhalten.

Weiterhin lassen sich die β-Oxosulfenylverbindungen der Formel X durch
Umsetzung von Ketonen der allgemeinen Formel XI 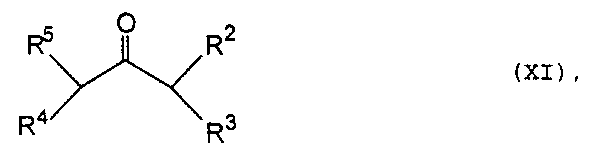 worin
R² - R⁵ die bei Formel I angegebene Bedeutung besitzen, mit
Verbindungen der allgemeinen Formel XIII

S₂Y₂ (XIII),

worin Y für Cl, Br und/oder J steht,
unter Ausschluß eines Zusatzes von Lösungsmittel und weiterer Lewis-Säuren und nachfolgender Umsetzung mit

Y₂

worin Y die oben genannte Bedeutung besitzt, erhalten.

Im Gegensatz zu den aus dem Stand der Technik bekannten Verfahren kann damit im Rahmen der Erfindung die Reaktion zur Herstellung der β-Oxosulfenylverbindungen der Formel X ohne Lösungsmittel und ohne Zusatz einer Lewis-Säure durchgeführt werden, wodurch die in der Literatur angegebene Ausbeuten erheblich gesteigert werden konnten. Weiterhin von besonders großem Vorteil ist die Tatsache, daß die bei der Herstellung der Oxosulfenylverbindungen der Formel II nach dem Verfahren der Erfindung anfallende Reaktionsmischung ohne weitere komplizierte Aufarbeitung einfach durch Auflösen in einer als Lösungsmittel geeigneten organischen Verbindung weiterverarbeitet werden kann.

Erfindungsgemäß ist es des weiteren bevorzugt, für die Umsetzung der Ketone der allgemeinen Formel XI Schwefeldichlorid als Verbindung der allgemeinen Formel XII bzw. Dischwefeldichlorid der allgemeinen Formel XIII und Chlor als Y₂ zur Spaltung auszuwählen. Diese Schwefelverbindungen sind sicher und einfach handhabbar und lassen die Herstellung der erfindungsgemäß zu zyklisierenden β-Oxosulfenylverbindungen der Formel X in hoher Ausbeute zu.

Die nachstehenden Beispiele dienen zur Erläuterung der Erfindung:

### Beispiel 1

### Herstellung von 2-(2,4-Dimethyl-3-oxo-pentyl)sulfenylchlorid = Verbindung X mit R² - R⁵ = Methyl und Y = Cl;

In einem 1 l-Dreihalskolben mit Tropftrichter, Thermometer und Vakuumanschluß werden 428 ml (3 mol) Diisopropylketon vorgelegt. Langsam werden nun 0,25 Äquivalente Schwefeldichlorid hinzugefügt. Nachdem die Reaktion angesprungen ist (Temperaturanstieg) werden unter Eiskühlung die restliche 0,75 Äquivalente Schwefeldichlorid so hinzugefügt, daß die Temperatur 30 °C nicht übersteigt. Nach Abnahme der Hydrogenchloridentwicklung wird nach 30 min weitergerührt und dann restliches Hydrogenchlorid bei 20 mbar im Wasserstrahlvakuum entfernt. Man erhält mit > 90 % Ausbeute das Sulfenylchlorid.

### Beispiel 2

### Herstellung des 2,2,4,4-Tetramethylthietanons = Verbindung VII mit R² - R⁵ = Methyl;

1,8 g (10 mmol) des in Beispiel 1 erhaltenen Sulfenylchlorids werden in 140 ml Tetrahydrofuran gelöst und unter Eiskühlung zu einer Lösung von 1,23 g (11 mmol) Kaliumtertiärbutylat in 50 ml Tetrahydrofuran getropft. Man läßt die Mischung noch ca. 1 Stunde rühren. Die Aufarbeitung erfolgt durch Ansäuern mit 1 M Salzsäure auf pH = 3. Nach Zugabe von 50 ml Wasser wird die Lösung 3 mal mit 100 ml Diethylether extrahiert. Die gesammelten Etherphasen werden über Magnesiumsulfat getrocknet. Nach Filtration wird die Ausbeute mittels HPLC auf 91 % bestimmt.

### Beispiel 3

### Herstellung des Thietanonoxims der allgemeinen Formel IX mit R² - R⁵ = Methyl, X = OH;

7,2 g (50 mmol) des Thietanons aus Beispiel 2 (R² - R⁵ = Methyl) werden mit 6,9 g (100 mmol) Hydroxylaminhydrochlorid und 6,8 g (50 mmol) Kaliumdihydrogenphosphat in 200 ml Methanol suspendiert und in einem Autoklaven für 12 h auf 120 °C erhitzt. Anschließend werden zur Suspension 100 ml Wasser hinzugefügt, und das Methanol wird im Vakuum entfernt. Das Oxim kristallisiert aus, wird abfiltriert und getrocknet. Man erhält 7,2 g (90,5 %) des Oxims.

### Beispiel 4

### Herstellung des Thietanamins der allgemeinen Formel VI mit R² - R⁵ = Methyl;

790 mg (5 mmol) des Oxims aus Beispiel 3 (R² - R⁵ = Methyl, X = OH) werden in 20 ml Diglyme gelöst und mit 190 mg (5 mmol) Natriumborhydrid versetzt. Dann werden bei -15 °C 0,6 ml einer Lösung aus 48 ml Diglyme und 12 ml konz. Schwefelsäure zugetropft. Man erwärmt vorsichtig auf 110 °C und beläßt die Suspension bei dieser Temperatur für ca. 4 h. Anschließend fügt man 10 ml 20 %iger KOH-Lösung hinzu und erhitzt für weitere 60 min auf 100 °C. Diese Lösung wird ohne weitere Aufarbeitung zur Weiterverarbeitung benutzt. Die mittels HPLC bestimmte Ausbeute an Thietanamin beträgt 90,5 % bezogen auf das eingesetzte Oxim gemäß Beispiel 3.

### Beispiel 5

### Herstellung von 2-(2,4-Dimethyl-3-oxo-pentyl)sulfenylchlorid (R¹ - R⁴ in Formel II = Methyl, X = Cl)

In einem 1 1 Dreihalskolben mit Tropftrichter und Thermometer sowie Destillationsaufsatz bestückt werden 4 Äquiv. DIPK vorgelegt. Bei < 10 °C wird 1 Äquiv. S₂Cl₂ hinzugefügt. Nach erfolgter Reaktion entfernt man überschüssiges DIPK im Vakuum und leitet anschließend Chlor bis zu Sättigung ein. Die leichtflüchtigen Verbindungen werden dann im Vakuum entfernt und das Sulfenylchlorid kann in die folgende Umsetzung eingesetzt werden.

### Beispiel 6

### Herstellung des 2,2,4,4-Tetramethylthietanons (R¹ - R⁴ in Formel I = Methyl)

Das Sulfenylchlorid aus Beispiel 5 wird in eine Lösung aus 1.5 Äquiv. (bezogen aufs Sulfenylchlorid) Natriummethylat in Methanol getropft. Anschließend erhitzt man 3 - 4 h am Rückfluß. Man gibt in der Hitze 1/5-tel des Volumens an Wasser hinzu, läßt abkühlen und filtriert ab. In > 80 % Ausbeute erhält man das Keton als farblosen, campherartig riechenden Feststoff.

### Beispiel 7

A) Kopplung von D-Ala-thietanamid der allgemeinen Formel II mit Z-Oxazolidinon der allgemeinen Formel III (R₆ = R₇ = H, R₁ = Z-Gruppe)
   26,7 g (100 mmol) N-Benzyloxycarbonylasparaginsäure werden mit 6.0 g (200 mmol) Paraformaldehyd und 0.38 g (2 mmol) p-Toluolsulfonsäure in 500 ml Toluol gelöst und 1 h unter Rückfluß bei 110 °C am Wasserabscheider erhitzt. Anschließend wird die Lösung mit 21,6 g (100 mmol) D-Ala-thietanamid der allgemeinen Formel II und 14 ml (100 mmol) Triethylamin versetzt und für 6 h auf 60 °C erhitzt. Anschließend wird die Lösung mit 100 ml 1 N Salzsäure und anschließend mit 100 ml Wasser gewaschen. Man trocknet die org. Phase über Magnesiumsulfat. Nach Filtration engt man im Vakuum ein und erhält in 85 %iger Ausbeute das Z-geschützte Alitam der allgemeinen Formel I (R₁ = Z).
B) Kopplung des D-Ala-thietanamins der allgemeinen Formel II mit Oxazolidinon der allgemeinen Formel III (R₁ = H, R_{6,7} = CF₃):
   13,3 g (100 mmol) L-Asp werden in 300 ml DMF suspendiert. In die Suspension leitet man einen leichten Strom von Hexafluoraceton. Nachdem 2,5 bis 3 Äquiv. in die Lösung geleiten wurden, rührt man, bis alles in Lösung gegangen ist. Anschließend wird das gebildete Hexafluoracetonhydrat im Vakuum entfernt und in einer bestimmten Menge Wasser aufgefangen. Sodann entfernt man das Lösungsmittel im Vakuum. Der verbleibende ölige Rest wird in 100 ml THF gelöst und langsam mit einer Lösung von 21,6 g (100 mmol) D-Ala-thietanamid in 100 ml THF bei RT versetzt. Man rührt 12 h, entfernt das sich bildende Hexafluoraceton im Vakuum und leitet es ebenfalls in eine bestimmte Menge Wasser ein. Die wäßrigen Hexafluoraceton-Lösungen können anschließend mit konz. Schwefelsäure wieder entwässert werden. Den nach Abzug des Lösungsmittels verbleibenden Rückstand arbeitet man wie in US-PS 4,411,925 beschrieben auf. Man erhält in 80 %iger Ausbeute Alitam.
C) Kopplung des D-Ala-thietanamins der allgemeinen Formel II mit Oxazolidionon der allgemeinen Formel III (R₁ = H, R₆ = H, R₇ = CCl₃):
   1,33 g (10 mmol) L-Asp werden in 3 ml DMSO suspendiert und mit 1,95 ml (20 mmol) Chloral versetzt. Man rührt die Lösung für 4 h bei RT. Dann kühlt man die Lösung auf -15 °C und versetzt mit 50 ml THF. Langsam läßt man bei dieser Temperatur eine Lösung von 21,6 g (100 mmol) D-Ala-thietanamid in 50 ml THF in die kalte Mischung tropfen. Man rührt die Mischung 12 h bei dieser Temperatur, filtriert und reinigt den Rückstand säulenchromatographisch auf. Man erhält in 80 %iger Ausbeute Alitam.
   Weitere Vorteile und Ausführungsformen der Erfindung ergeben sich aus den nachfolgenden Patentansprüchen.

## Patentansprüche

1. Verfahren zur Herstellung von L-Aspartyl-D-Alanin-N-(thietan-3-yl)-amiden der allgemeinen Formel I 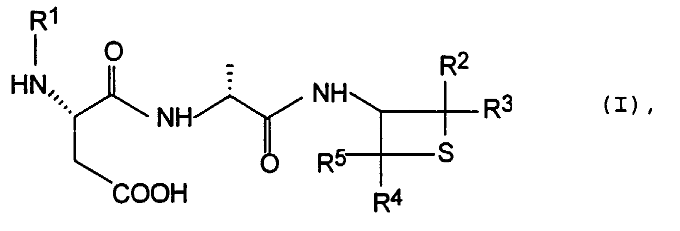 worin
R¹ für H oder eine selektiv abspaltbare Schutzgruppe steht und
R² - R⁵ unabhängig voneinander gleich oder verschieden H oder C₁-C₄-Alkyl, linear oder verzweigt, bedeuten,
durch Umsetzung von D-Alanin-thietanamiden der allgemeinen Formel II 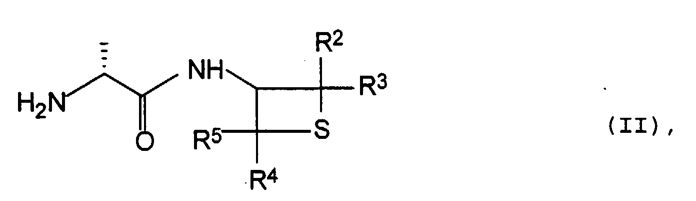 worin
R² - R⁵ die bei Formel (I) angegebene Bedeutung besitzen,
mit
Oxazolidinonverbindungen der allgemeinen Formel III 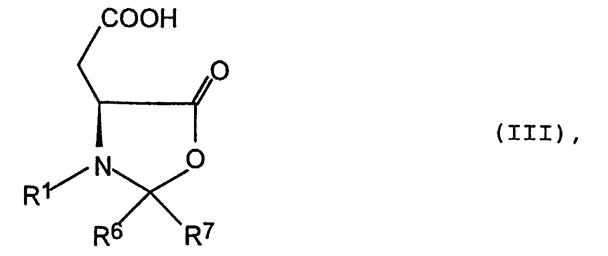 worin
R¹ die bei Formel I angegebene Bedeutung besitzt und R⁶ und R⁷ unabhängig voneinander gleich oder verschieden für H, C₁-C₄-Alkyl, linear oder verzweigt, C₁-C₄-Haloalkyl, linear oder verzweigt, Aryl oder einen die Carbonylgruppe aktivierenden Rest stehen, in einem inerten organischen Lösungsmittel.

2. Verfahren nach Anspruch 1,
**dadurch gekennzeichnet,**
daß eine Verbindung der Formel III mit R¹ = H, R⁷ = CF₃ und R⁶ = CF₃ eingesetzt wird, die durch Umsetzung von L-Asparaginsäure mit Hexafluoraceton erhalten wird.

3. Verfahren nach Anspruch 2,
**dadurch gekennzeichnet**,
daß die Umsetzung von L-Asparaginsäure mit Hexafluoraceton in DMF als Lösungsmittel durchgeführt wird und überschüssiges Hexafluoraceton und/oder Hexafluoracetonhydrat vollständig zurückgewonnen werden.

4. Verfahren nach Anspruch 3,
**dadurch gekennzeichnet**,
daß das resultierende Bis-(2,2-trifluor-4-carboxymethyl)-1,3-oxazolidin-5-on ohne weitere Reinigung mit einer Verbindung der allgemeinen Formel II gekoppelt wird.

5. Verfahren nach Anspruch 1,
**dadurch gekennzeichnet**,
daß eine Verbindung der Formel III mit R¹ = H und R⁶ oder R⁷ = CCl₃ eingesetzt wird, die durch Umsetzung von L-Asparaginsäure mit Chloral erhalten wird.

6. Verfahren nach Anspruch 1,
**dadurch gekennzeichnet**,
daß eine Verbindung der Formel III eingesetzt wird, in der R¹ für eine N-Schutzgruppe steht, wobei die Verbindung der Formel III durch Umsetzung von N-geschützter L-Asparaginsäure der allgemeinen Formel IV 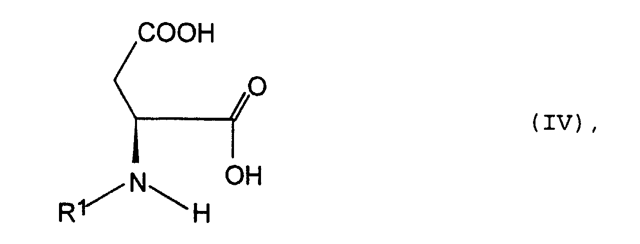 worin
R¹ für Boc, Z, TFA, Aloc, Teoc, Formyl, Tosyl, Fmoc oder Moc steht,
mit Carbonylverbindungen, vorzugsweise Aldehyden, besonders bevorzugt Formaldehyd oder einer bei der Umsetzung Formaldehyd erzeugenden Vorstufe, erhalten wird.

7. Verfahren nach Anspruch 6,
**dadurch gekennzeichnet**,
daß das Umsetzungsprodukt aus IV und Formaldehyd ohne weitere Isolierung und Aufreinigung zur Kopplung mit II eingesetzt wird.

8. Verfahren nach Anspruch 6 oder 7,
**dadurch gekennzeichnet**,
daß die Umsetzung der N-geschützten L-Asparaginsäure mit Paraformaldehyd in Toluol oder MiBK als Lösungsmittel durchgeführt wird.

9. Verfahren nach einem der Ansprüche 6 bis 8,
**dadurch gekennzeichnet,**
daß als Verbindung der Formel IV eine mit der Boc- oder Formyl-Gruppe N-geschützte L-Asparaginsäure eingesetzt wird.

10. Verfahren nach Anspruch 1,
**dadurch gekennzeichnet**,
daß die Verbindungen der allgemeinen Formel II durch Kopplung von D-Alanin-Verbindungen der allgemeinen Formel V 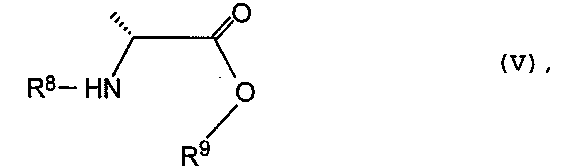 worin
R⁸ eine selektiv abspaltbare N-Schutzgruppe bedeutet und R⁹ ein aus der Peptidchemie bekannter, die Carbonylgruppe aktivierender Rest ist,
mit
Thietanaminen der allgemeinen Formel VI 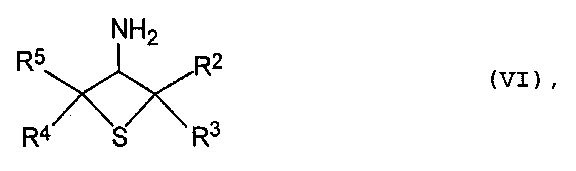 worin
R² - R⁵ die bei Formel I angegebene Bedeutung besitzen,
erhalten werden.

11. Verfahren nach Anspruch 10,
**dadurch gekennzeichnet**,
daß Thietanamine der allgemeinen Formel VI durch Kondensation von
Thietanonen der allgemeinen Formel VII 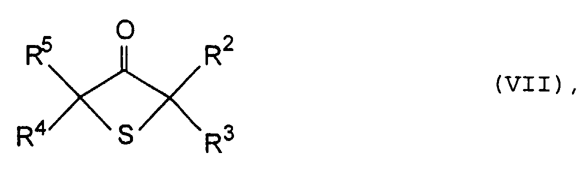 worin
R² - R⁵ die bei Formel I angegebene Bedeutung besitzen, mit Aminderivaten der allgemeinen Formel VIII
NH₂-X (VIII),
worin X für OH, O-Alkyl, O-Aryl, O-Acyl oder NHR¹⁰ steht und R¹⁰ = C₁-C₄-Alkyl, Aryl, Aralkyl, Tosyl, Mesyl oder Acyl ist, oder deren Salze, wie beispielsweise Hydrohalogenide, Phosphate, Citrate, Acetate oder Sulfate,
zu Thietaniminen der allgemeinen Formel IX 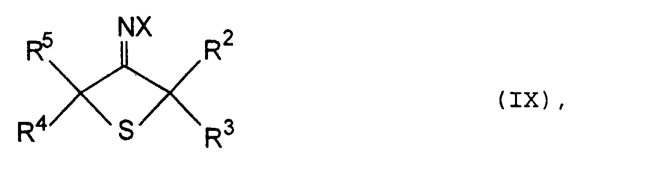
worin
R² - R⁵ die bei Formel I angegebene Bedeutung besitzen und X die Bedeutung wie in Formel (VIII) hat, und anschließende Reduktion der erhaltenen
Thietaniminderivate,
erhalten werden.

12. Verfahren nach Anspruch 11,
**dadurch gekennzeichnet**,
daß die Kondensation im Autoklaven bei einer Temperatur von > 80 °C, bevorzugt > 100 °C, in Gegenwart eines Salzes einer Säure, deren pKs-Wert zwischen 1 und 4 liegt, und die Reduktion mit Natrium- oder Lithiumborhydrid und einem Aktivator durchgeführt werden.

13. Verfahren nach Anspruch 12,
**dadurch gekennzeichnet**,
daß als Salz der Säure, deren pKs-Wert zwischen 1 und 4 liegt, ein Säureadditionssalz des Amins der allgemeinen Formel VIII verwendet wird.

14. Verfahren nach Anspruch 12 oder 13,
**dadurch gekennzeichnet**,
daß als Aktivator bei der Reduktion Jod, Chlorwasserstoff oder Schwefelsäure eingesetzt wird.

15. Verfahren nach einem der Ansprüche 11 - 14,
**dadurch gekennzeichnet,**
daß die Reduktion in einem Lösungsmittel mit Etherstruktur durchgeführt wird.

16. Verfahren nach einem der Ansprüche 11 - 15,
**dadurch gekennzeichnet**,
daß die Reduktion bei Temperaturen > 80 °C, bevorzugt > 110 °C, durchgeführt wird.

17. Verfahren nach einem der Ansprüche 11 - 16,
**dadurch gekennzeichnet**,
daß die Reduktion mit dem System Natriumborhydrid/Schwefelsäure durchgeführt wird.

18. Verfahren nach einem der Ansprüche 11 - 17,
**dadurch gekennzeichnet**,
daß zur Kondensation Hydroxylamin, dessen Hydrochlorid oder -sulfat bzw. -phosphat oder Dihydrosulfat eingesetzt wird.

19. Verfahren nach einem der Ansprüche 11-18,
**dadurch gekennzeichnet**,
daß Verbindungen umgesetzt werden, worin R² - R⁵ jeweils Methyl sind.

20. Verfahren nach einem der Ansprüche 11-19,
**dadurch gekennzeichnet**,
daß die Thietanone der allgemeinen Formel VII durch Zyklisierung von β-Oxosulfenylen der allgemeinen Formel X 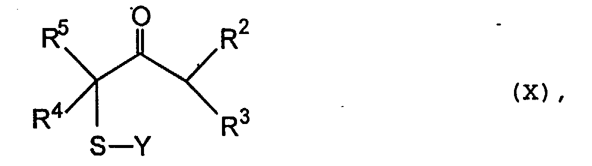 worin
R² - R⁵ die bei Formel I angegebene Bedeutung besitzen und Y für Cl, Br, J steht,
in einem organischen Lösungsmittel unter Einfluß einer Base, erhalten werden.

21. Verfahren nach Anspruch 20,
**dadurch gekennzeichnet**,
daß als organisches Lösungsmittel ein Ether und als Base Kaliumtertiärbutylat verwendet wird.

22. Verfahren nach Anspruch 20,
**dadurch gekennzeichnet,**
daß als Lösungsmittel ein (C₁-C₆) Alkohol und als Base das entsprechende Alkalialkoholat verwendet wird.

23. Verfahren nach Anspruch 22,
**dadurch gekennzeichnet**, daß als Lösungsmittel Methanol und als Base Natriummethanolat verwendet wird.

24. Verfahren nach einem der Ansprüche 11-23,
**dadurch gekennzeichnet**,
daß die β-Oxosulfenylverbindungen der Formel X durch Umsetzung von Ketonen der allgemeinen Formel XI 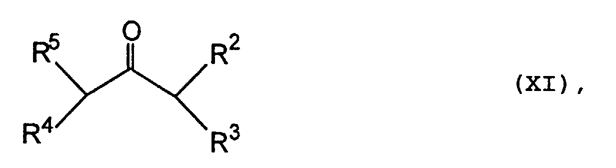 worin
R² - R⁵ die bei Formel I angegebene Bedeutung besitzen, mit
Verbindungen der allgemeinen Formel XII
SY₂ (XII),
worin Y für Cl, Br und/oder J steht,
unter Ausschluß eines Zusatzes von Lösungsmittel und weiterer Lewis-Säuren erhalten werden.

25. Verfahren nach einem der Ansprüche 11-23,
**dadurch gekennzeichnet**,
daß die β-Oxosulfenylverbindungen der Formel X durch Umsetzung von Ketonen der allgemeinen Formel XI 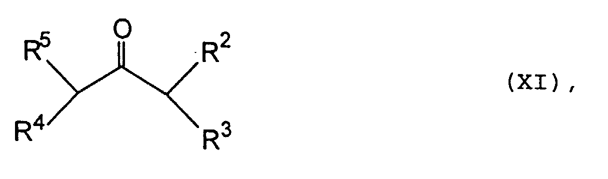 worin
R² - R⁵ die bei Formel I angegebene Bedeutung besitzen, mit
Verbindungen der allgemeinen Formel XIII
S₂Y₂ (XIII),
worin Y für Cl, Br und/oder J steht,
unter Ausschluß eines Zusatzes von Lösungsmittel und weiterer Lewis-Säuren und nachfolgender Umsetzung mit
Y₂
worin Y die oben genannte Bedeutung besitzt, erhalten werden.

26. Verfahren nach Anspruch 24 oder 25,
**dadurch gekennzeichnet**,
daß die resultierenden β-Oxosulfenylverbindungen der Formel X ohne Aufarbeitung in einem Ether gelöst und mit einer Base unter Erhalt der Thietanone gemäß Formel VII zyklisiert werden.

27. Verfahren nach einem der Ansprüche 11-26,
**dadurch gekennzeichnet**,
daß Verbindungen umgesetzt werden, in denen die Reste R² - R⁵ alle jeweils für eine Methylgruppe stehen.

28. Verfahren nach einem der Ansprüche 11-27,
**dadurch gekennzeichnet**,
daß Verbindungen umgesetzt werden, in denen Y für Cl steht.

## Claims

1. A process for preparing L-aspartyl-D-alanine-N-(thietan-3-yl) amides of the general formula I 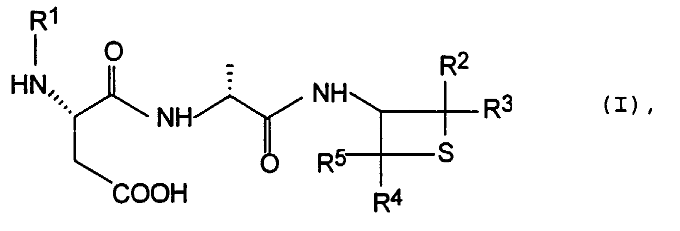 in which
R¹ represents H or a selectively separable protective group and
R² - R⁵ are identical or different and, independently, represent H or a linear or branched C₁-C₄-alkyl group,
by reacting D-alanine-thietane amides of the general formula II 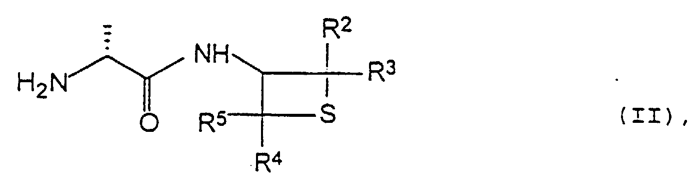 in which
R² - R⁵ are defined in the same way as for formula (I),
with
oxazolidinone compounds of the general formula III 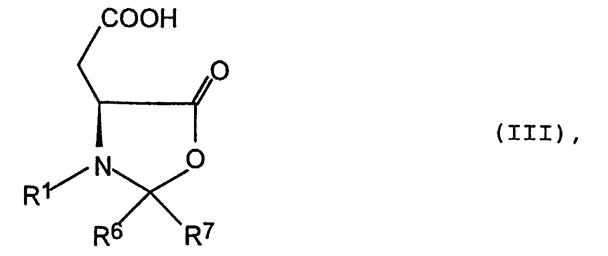 in which
R¹ is defined in the same way as for formula I and R⁶ and R⁷ are identical or different and, independently, represent H, a linear or branched C₁-C₄-alkyl, linear or branched C₁-C₄-haloalkyl or aryl group or a group which activates the carbonyl group, in an inert organic solvent.

2. A process according to Claim 1,
characterised in that
a compound of the formula III in which R¹ = H, R⁷ = CF₃ and R⁶ = CF₃ is used, which is obtained by reacting L-aspartic acid with hexafluoroacetone.

3. A process according to Claim 2,
characterised in that
the reaction of L-aspartic acid with hexafluoroacetone is performed in DMF as solvent and excess hexafluoroacetone and/or hexafluoroacetone hydrate is completely recovered.

4. A process according to Claim 3,
characterised in that
the resulting bis-(2,2-trifluoro-4-carboxymethyl)-1,3-oxazolidin-5-one is coupled with a compound of the general formula II without further purification.

5. A process according to Claim 1,
characterised in that
a compound of the formula III with R¹ = H and R⁶ or R⁷ = CCl₃ is used, which is obtained by reacting L-aspartic acid with chloral.

6. A process according to Claim 1,
characterised in that
a compound of the formula III is used, in which R¹ represents a N-protective group, wherein the compound of the formula III is obtained by reacting N-protected L-aspartic acid of the general formula IV 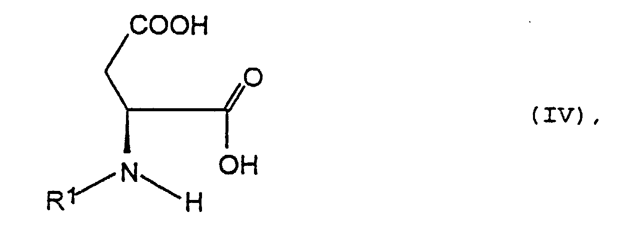 in which
R¹ represents Boc, Z, TFA, Aloc, Teoc, formyl, tosyl, Fmoc or Moc,
with carbonyl compounds, preferably aldehydes, in particular formaldehyde or a precursor which produces formaldehyde during the reaction.

7. A process according to Claim 6,
characterised in that
the reaction product of IV and formaldehyde is used to couple with II without further isolation or purification.

8. A process according to Claim 6 or 7,
characterised in that
the reaction of N-protected L-aspartic acid with paraformaldehyde is performed in toluene of MiBK as solvent.

9. A process according to one of Claims 6 to 8,
characterised in that
a L-aspartic acid which is N-protected with a Boc or formyl group is used as a compound of the formula IV.

10. A process according to Claim 1,
characterised in that
the compounds of the general formula II are obtained by coupling D-alanine compounds of the general formula V 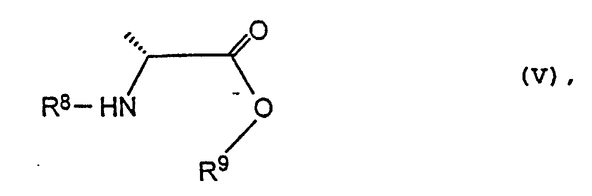 in which
R⁸ represents a selectively separable N-protective group and R⁹ is a group known from peptide chemistry which activates the carbonyl group,
with thietanamines of the general formula VI 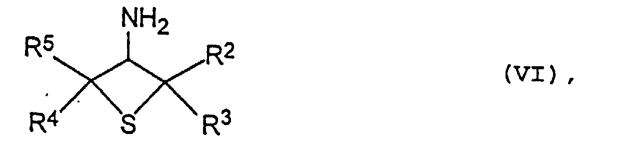 in which
R² - R⁵ are defined in the same way as for formula I.

11. A process according to Claim 10,
characterised in that
thietanamines of the general formula VI are obtained by condensation of thietanones of the general formula VII 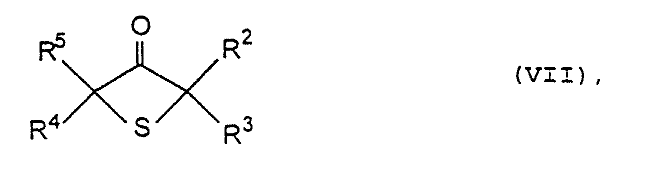 in which
R² - R⁵ are defined in the same way as for formula I, with amine derivatives of the general formula VIII
NH₂-X (VIII),
in which X represents OH, O-alkyl, O-aryl, O-acyl, or NHR¹⁰ and R¹⁰ = C₁-C₄-alkyl, aryl, aralkyl, tosyl, mesyl or acyl, or their salts, such as for example hydrohalides, phosphates, citrates, acetates or sulfates,
to give thietanimines of the general formula IX 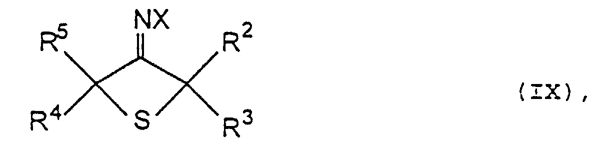
in which
R² - R⁵ are defined in the same way as for formula I and X is defined in the same way as for formula (VIII), followed by reduction of the thietanimine derivatives obtained.

12. A process according to Claim 11,
characterised in that
condensation is performed in autoclaves at a temperature of > 80°C, preferably > 100°C, in the presence of a salt of an acid with a pKa value between 1 and 4, and reduction is performed with sodium or lithium borohydride and an activator.

13. A process according to Claim 12,
characterised in that
an acid addition salt of the amine of the general formula VIII is used as a salt of an acid with a pKa value between 1 and 4.

14. A process according to Claim 12 or 13,
characterised in that
iodine, hydrogen chloride or sulfuric acid is used as an activator during reduction.

15. A process according to one of Claims 11 - 14,
characterised in that
reduction is performed in a solvent with an ether structure.

16. A process according to one of Claims 11 - 15,
characterised in that
reduction is performed at temperatures > 80°C, preferably > 110°C.

17. A process according to one of Claims 11 - 16,
characterised in that
reduction is performed using the sodium borohydride/sulfuric acid system.

18. A process according to one of Claims 11 - 17,
characterised in that
hydroxylamine, its hydrochloride or sulfate or phosphate or dihydrosulfate is used for condensation.

19. A process according to one of Claims 11 - 18,
characterised in that
compounds in which R² - R⁵ each represent methyl are used in the reactions.

20. A process according to one of Claims 11 - 19,
characterised in that
thietanones of the general formula VII are obtained by cyclisation of b-oxosulfenyl compounds of the general formula X 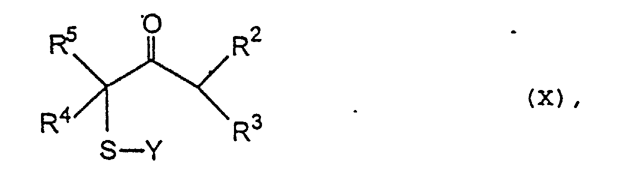 in which
R² - R⁵ are defined in the same way as for formula I and Y represents Cl, Br or I,
in an organic solvent under the effect of a base.

21. A process according to Claim 20,
characterised in that
an ether is used as organic solvent and potassium tertiary butylate is used as base.

22. A process according to Claim 20,
characterised in that
a (C₁-C₆)-alcohol is used as solvent and the corresponding alkali metal alcoholate is used as base.

23. A process according to Claim 22,
characterised in that
methanol is used as solvent and sodium methanolate is used as base.

24. A process according to one of Claims 11 - 23,
characterised in that
β-oxosulfenyl compounds of the formula X are obtained by reacting ketones of the general formula XI 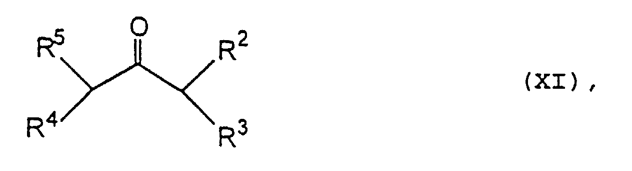 in which
R² - R⁵ are defined in the same way as for formula I, with
compounds of the general formula XII
SY₂ (XII),
in which Y represents Cl, Br and/or I,
without the addition of a solvent or other Lewis acids.

25. A process according to one of Claims 11 - 23,
characterised in that
β-oxosulfenyl compounds of the formula X are obtained by reacting ketones of the general formula XI 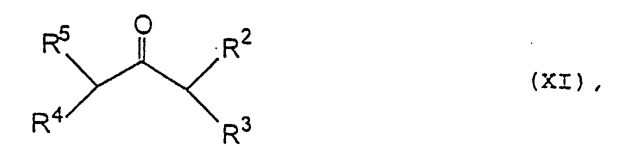 in which
R² - R⁵ are defined in the same way as for formula I, with
compounds of the general formula XIII
S₂Y₂ (XIII),
in which Y represents Cl, Br and/or I,
without the use of a solvent or other Lewis acids and subsequent reaction with
Y₂
in which Y is defined as above.

26. A process according to Claim 24 or 25,
characterised in that
the resulting β-oxosulfenyl compounds of the formula X, without working up, are dissolved in an ether and are cyclised with a base to give thietanones in accordance with formula VII.

27. A process according to one of Claims 11 - 26,
characterised in that
compounds are reacted in which all the groups R² - R⁵ each represent a methyl group.

28. A process according to one of Claims 11 - 27.
characterised in that
compounds are reacted in which Y represents Cl.

## Revendications

1. Procédé de fabrication de L-aspartyl-D-alanine-N-(thiétan-3-yl)-amides de formule générale I 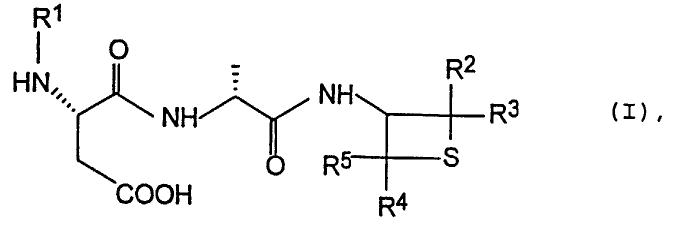 dans laquelle
R¹ représente H ou un groupe protecteur sélectivement séparable et
R²-R⁵ représentent indépendamment l'un de l'autre, de manière identique ou différente, H ou un alkyle en C₁ à C₄, linéaire ou ramifié,
par réaction de D-alanine-thiétanamides de formule générale II 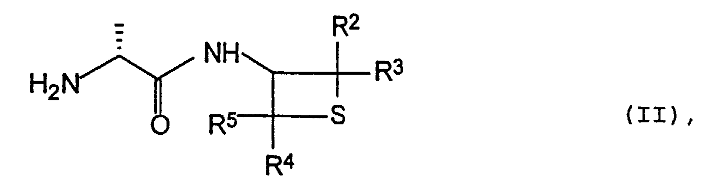 dans laquelle
R²-R⁵ ont la signification indiquée dans la formule I, avec
des compositions d'oxazolidinone de formule générale III 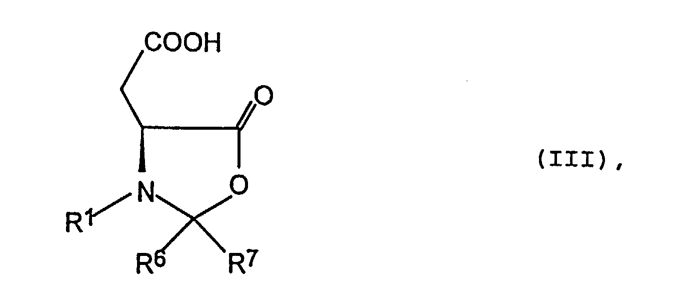 dans laquelle
R¹ a la signification indiquée dans la formule I, et
R⁶ et R⁷ représentent indépendamment l'un de l'autre, de manière identique ou différente, H, un alkyle en C₁ à C₄, linéaire ou ramifié, un halo-alkyle en C₁ à C₄, linéaire ou ramifié, un aryle ou un radical actif dans le groupe carbonyle, dans un solvant organique inerte.

2. Procédé selon la revendication 1,
caractérisé en ce qu'
on utilise un composé de formule III dans laquelle R¹ = H, R⁷ = CF₃, R⁶ = CF₃, que l'on obtient par réaction d'acide L-asparagine avec l'hexafluoracétone.

3. Procédé selon la revendication 2,
caractérisé en ce qu'
on conduit la réaction de l'acide L-asparagine avec l'hexafluoracétone dans le DMF comme solvant et en ce qu'on récupère entièrement l'excès d'hexafluoracétone et/ou d'hexafluoracétone hydratée.

4. Procédé selon la revendication 3,
caractérisé en ce qu'
on couple la bis-(2,2-trifuloro-4-carboxylméthyl)-1,3-oxazolidin-5-one résultante sans autre purification avec un composé de formule générale II.

5. Procédé selon la revendication 1,
caractérisé en ce qu'
on utilise un composé de formule III dans laquelle R¹ = H et R⁶ ou R⁷ = CCl₃, que l'on obtient par réaction d'acide L-asparagine avec du chloral.

6. Procédé selon la revendication 1,
caractérisé en ce qu'
on utilise un composé de formule III dans laquelle R¹ représente un groupe N-protecteur, dans lequel on obtient le composé de formule III par réaction d'acide L-asparagine L-protégé de formule générale IV 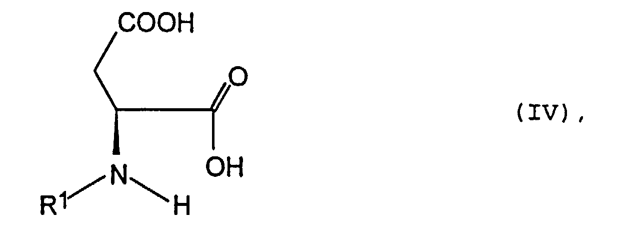 dans laquelle
R¹ représente Boc, Z, TFA, Aloc, Teoc, un formyle, un tosyle, Fmoc ou Moc,
avec des composés carbonyles, de préférence des aldéhydes, de façon particulièrement préférée le formaldéhyde ou un précurseur produit dans la réaction du formaldéhyde.

7. Procédé selon la revendication 6,
caractérisé en ce qu'
on utilise le produit de la réaction de IV et de formaldéhyde sans autre isolement et purification pour le couplage avec II.

8. Procédé selon la revendication 6 ou 7,
caractérisé en ce qu'
on conduit la réaction de l'acide L-asparagine n-protégé avec du paraformaldéhyde dans le toluène ou la méthyl-isobutyl-cétone (MiBK) comme solvant.

9. Procédé selon l'une des revendications 6 à 8,
caractérisé en ce qu'
on utilise comme composé de formule IV un acide L-asparagine N-protégé avec le groupe Boc ou le groupe formyle.

10. Procédé selon la revendication 1,
caractérisé en ce qu'
on obtient les composés de formule générale II par couplage de composés de D-alanine de formule générale V 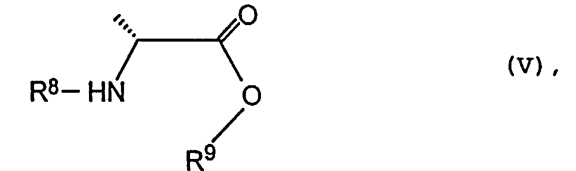 dans laquelle
R⁸ représente un groupe protecteur de N sélectivement séparable et R⁹ un radical activant le groupe carbonyle, connu en chimie des peptides, avec
des thiénamines de formule générale VI 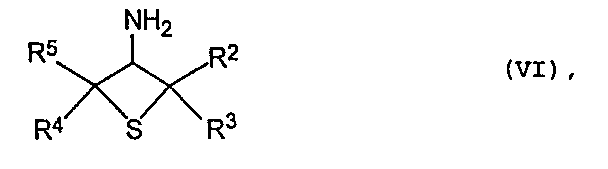 dans laquelle R²-R⁵ ont la signification indiquée dans la formule I.

11. Procédé selon la revendication 10,
caractérisé en ce qu'
on obtient les thiétanamines de formule générale VI par condensation de thiétanones de formule générale VII 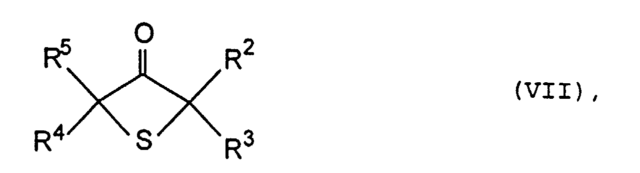 dans laquelle R²-R⁵ ont la signification indiquée dans la formule I,
avec des dérivés amines de formule générale VIII
NH₂-X (VIII),
dans laquelle X représente OH, un O-alkyle, un O-aryle, un O-acyle ou NHR¹⁰ et R¹⁰ = alkyle en C₁ à C₄, aryle, aralkyle, tosyle, mésyle ou acyle, ou de leurs sels comme par exemple les hydrohalogénures, les phosphates, les citrates, les acétates ou les sulfates,
pour donner des thiétanimines de formule générale XI 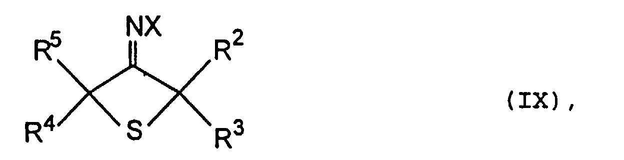 dans laquelle R²-R⁵ ont la signification indiquée dans la formule I et X a la même signification que dans la formule (VIII),
puis par réduction des dérivés de thiétanamines obtenues.

12. Procédé selon la revendication 11,
caractérisé en ce qu'
on conduit la condensation à l'autoclave à une température supérieure à 80°C, de préférence supérieure à 100°C, en présence d'un sel, d'un acide, dont la valeur de pKs se situe entre 1 et 4, et en ce qu'on effectue la réduction avec du borohydrure de sodium ou de lithium et un activateur.

13. Procédé selon la revendication 12,
caractérisé en ce qu'
on utilise comme sel de l'acide dont la valeur de pKs se situe entre 1 et 4 un sel d'addition de l'amine de formule générale VIII.

14. Procédé selon la revendication 12 ou 13,
caractérisé en ce qu'
on utilise comme activateur lors de la réduction l'iode, l'acide chlorhydrique ou l'acide sulfurique.

15. Procédé selon l'une des revendications 11-14,
caractérisé en ce qu'
on conduit la réduction dans un solvant à structure d'éther.

16. Procédé selon l'une des revendications 11-15,
caractérisé en ce qu'
on conduit la réduction à des températures supérieures à 80°C, de préférence supérieures à 110°C.

17. Procédé selon l'une des revendications 11-16,
caractérisé en ce qu'
on conduit la réduction avec le système borohydrure de sodium/acide sulfurique.

18. Procédé selon l'une des revendications 11-17,
caractérisé en ce qu'
on utilise pour la condensation l'hydroxylamine, son chlorhydrate ou sulfate, ou selon les cas son phosphate ou dihydrosulfate.

19. Procédé selon l'une des revendications 11-18,
caractérisé en ce qu'
on fait réagir les composés dans lesquels R²-R⁵ représentent toujours un méthyle.

20. Procédé selon l'une des revendications 11-19,
caractérisé en ce qu'on obtient les thiétanols de formule générale VII par cyclisation de β-oxosulfényles de formule générale X 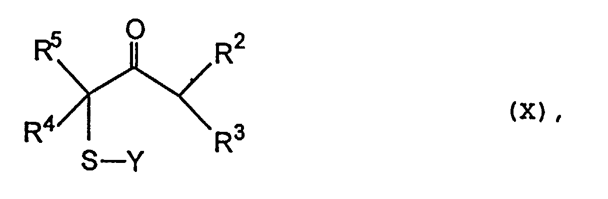 dans laquelle
R²-R⁵ ont la signification indiquée dans la formule I et Y représente Cl, Br, J,
dans un solvant organique sous l'influence d'une base.

21. Procédé selon la revendication 20,
caractérisé en ce qu'
on utilise comme solvant organique un éther et comme base le butylate tertiaire de potassium.

22. Procédé selon la revendication 20,
caractérisé en ce qu'
on utilise comme solvant un alcool en C₁ à C₆ et comme base l'alcoolate alcalin correspondant.

23. Procédé selon la revendication 22,
caractérisé en ce qu'
on utilise comme solvant le méthanol et comme base le méthanolate de sodium.

24. Procédé selon l'une des revendication 11-23,
caractérisé en ce qu'
on obtient les composés de β-oxosulfinyle de formule X par réaction de cétones de formule générale XI 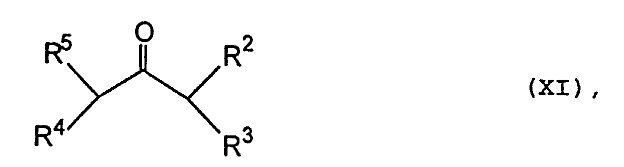 dans laquelle
R²-R⁵ ont la signification indiquée pour la formule I avec
des composés de formule générale XII
SY₂ (XII)
dans laquelle Y représente Cl, Br et/ou J,
à l'exclusion d'une addition de solvant et d'autres acides de Lewis.

25. Procédé selon l'une des revendications 11-23,
caractérisé en ce qu'
on obtient les composés de β-oxosulfényle de formule X par réaction de cétones de formule générale XI 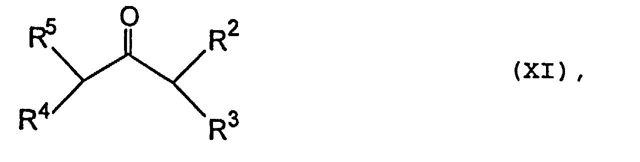 dans laquelle
R²-R⁵ ont la signification indiquée pour la formule I, avec
des composés de formule générale XIII
S₂Y₂ (XIII)
dans laquelle Y représente Cl, Br et/ou J,
à l'exclusion d'une addition de solvant et d'autres acides de Lewis, puis par réaction avec
Y₂
où Y a la signification mentionnée ci-dessus.

26. Procédé selon les revendications 24 ou 25,
caractérisé en ce qu'
on dissout les composés β-oxosulfényle de formule X résultants sans purification dans un éther et en ce qu'on les cyclise avec une base en obtenant les thiétanones selon la formule VII.

27. Procédé selon l'une des revendications 11-26,
caractérisé en ce qu'
on fait réagir des composés dans lesquels les radicaux R²-R⁵ représentent tous un groupe méthyle.

28. Procédé selon l'une des revendications 11-27
caractérisé en ce qu'
on fait réagir des composés dans lesquels Y représente Cl.
